# EUROPEAN PATENT SPECIFICATION

(11) **EP 1 338 843 B1**
(45) Date of publication and mention of the grant of the patent: **07.07.2010**
(21) Application number: 03075498.0
(22) Date of filing: 20.02.2003
(51) Int. Cl.: F17C 1/16, F17B 1/26

(54) **A membrane gas accumulator**
Membranspeicher für Gas
Accumulateur de gaz à membrane

(30) Priority: 21.02.2002 IT CR20020001
(43) Date of publication of application: 27.08.2003
(73) Proprietor: Ecomembrane S.R.L., 26030 Gadesco Pieve Delmona (CR) (IT)
(72) Inventor: Spedini, Luigi, 26100 Cremona (IT)
(74) Representative: Marcio', Paola

(56) References cited:
- EP-A- 0 333 698
- EP-A- 0 350 455
- FR-A- 2 353 790
- FR-A- 2 766 255

## Description

The present invention concerns a membrane gas accumulator, that may be used in particular for storing bio-gas generated by digestion of mud or sewage, or for the accumulation of gas exhaling from basins for the collection of liquids.

The membrane gas accumulators - also called membrane gasometers or dry gasometers or manostatic accumulators - usually comprise a first membrane that limits a gas accumulation chamber and a second membrane that creates a pressurizing chamber, usually an air pressurized chamber, adjacent to said gas accumulation chamber. One gas accumulator is disclosed in FR 2766255.

The second membrane partially limits the pressurized air chamber and protects the inner membrane from wind and rain, from atmospheric agents and from impacts with external bodies.

The accumulation chamber is connected to gas inlet and gas outlet pipes, or it is directly communicating with a basin for the collection of liquids or mud, from which the gas exhales.
The pressurizing chamber, instead, is connected to an auxiliary air fan or compressor, that allow to maintain said chamber at a determined pressure.

The pressure exerted by the pressurizing chamber onto the gas accumulation chamber allows to deliver gas at the desired pressure, according to the use of the gas itself.

The membranes are flexible, usually made of plastic material or plastic-coated fabric, e.g. PVC-coated fabric.

In a common kind of manostatic accumulators the first membrane forms a dome over a base surface impermeable to gas, and the second membrane forms an external dome which encloses the first one.
So, a central gas accumulation chamber, inside the first membrane, and a pressurizing chamber, comprised between the two membranes, are defined.

Said base surface may be, for example, a concrete basing or even another membrane fixed to the first membrane along its edge. If the first membrane forms a bottom closed chamber, the base surface is formed by part of the first membrane itself.

The base surface may also be the free surface of a liquid in which the edges of said first membrane are plunged. Said liquid may be water or a liquid contained in a collection basin from which said gas exhales, as already known from European patent no. EP 0 350 455 of the same inventor.

Accumulators of this type are realized with the shape of a spherical dome, a so-called "3/4 of a sphere" dome, or a cylindrical dome, the latter having substantially a rectangular or elliptical base surface.

There are also close-cylinder gas accumulators, which have a mainly horizontal development and in which the second membrane is externally connected or welded to the first membrane along two diametrically opposed edges, so as to form a pressurizing chamber adjacent to the gas accumulation chamber, along the whole length of the accumulator.

First of all, impermeability of the chambers is required in order to assure the functioning and the safety of the membrane gasometers. In fact, no gas leakage from the accumulation chamber to the air pressurizing chamber, nor gas or air leakage to the outside must occur.

Furthermore, the membranes must be hooked to the ground or to a fixed structure. The hooking must be resistant, reliable and such as not to tear or damage the membranes following to their movements, caused by filling and emptying of said chambers, as well as following to possible stress induced by wind or bad weather.

A further need is to know the filling degree of the gas accumulation chamber, for the managing and the adjusting of the accumulator during the normal functioning, as well as for safety reasons, so as to immediately detect gas leaks or other inconveniences.
As the second membrane externally encloses the first one, this is not possible by means of a simple visual control, but requires a special measuring device.

In the membrane gas accumulators used at present, the gas accumulation chambers and the air pressurizing chambers are separated only by a portion of the first membrane, and thus the seal between the chambers relies on the integrity of said first membrane. Furthermore, in the cylindrical or spherical accumulators, both the edge of the internal dome, formed by the first membrane, and the edge of the external dome, formed by the second membrane, must be sealed in an impermeable manner for maintaining the two chambers insulated.

If the first inner membrane forms a closed chamber, it may be hooked only in a mechanical way, e.g. with a portion of fabric fixed to the membrane and to fixed hooking means, or even with hooking ropes. Anyway, the hooking of the lower edge of the second membrane must be impermeable for insulating the air pressurized chamber from the outside.

A solution is known from European patent EP 0 333 698, that allows to seal the edge of a membrane to the base surface and, at the same time, to hook the membrane to the ground.

It mainly consists in locking the edge of the membrane with a certain force between two smooth plane surfaces acting as a seal, e.g. two metal plates or sections, tied to each other by a series of tie-rods at suitable distance. For increasing the safety of the mechanical fixing, the edge of the membrane also comprises a small holding rope which, in case of sliding of the edge of the membrane between the tightening surfaces, works as a stop and prevents the membrane from completely sliding away.

In closed-cylinder accumulators, on the other hand, the impermeability is given by the surfaces of the membranes, and the hooking to the ground is usually realized by means of external tie-rods fixed to said membranes and mainly obtained with elastic ropes or rigid ropes fixed to the ground and to the median plane of the accumulator.

For measuring the gas content, a common solution consists in measuring the distance between the top of the first membrane and the corresponding top of the second membrane.
In fact, the second membrane, being pressurized, substantially keeps always its shape, while the first membrane rises and lowers according the quantity of gas contained; consequently, the measuring of the distance between the top of the membranes gives an indication that is easily geometrically correlated to the gas content of the gas accumulation chamber.

A system used for such purpose consists in fixing to the top of the inner membrane a cable that unrolls from a spool fixed to the top of the outer membrane, and is provided with a torsion spring for keeping it always in tension.
While the cable rolls up and down following to the movements of the internal membrane that rises or lowers according to the pressure of the gas, a potentiometer indicates the rotation of the spool and thus the distance between the tops of the membranes.

Another system is known from French patent no. FR 2 766 255, based on a sufficiently heavy ballast resting on the top of the inner membrane, and a probe, usually an ultrasonic probe, fixed to the top of the outer membrane. Said probe measures the distance between itself and the ballast, that is the distance between the tops of the membranes.

All these mentioned solutions, of common use, for separating the chambers, for the fixed hooking to the ground and for the measuring of the contained gas volume, however, show some disadvantages.

A considerable problem consists in that, by means of above described solutions, the separation between the two chambers is given only by the first membrane and, possibly, by the sealed closing of its lower edge.

In case of fissures of the first membrane, even of very small dimensions, specially in correspondence of the connections between its different composing elements or in correspondence of the flanged connection, or even in case of imperfect sealing of the lower edge of said membrane, gas leaks from the gas accumulation chamber to the air pressurizing chamber may occur.
Such leaks always occur from the gas chamber to the air chamber, because gas pressure is equal to air pressure plus a pressure increase caused by the weight of the first membrane.
It must be considered that a minimum gas leakage into the air chamber may form an explosive mixture inside said chamber, with serious risks for the safety. Therefore, for a great advantage in terms of safety, it would be desirable that possible gas leaks from the accumulation chamber were directly led to the outside, instead of filtering into the pressurizing chamber. This is not possible in the present manostatic accumulators, so active safety systems are used, like a continuous washing of the pressurizing chamber with air exceeding the quantity requested for restoring the pressure. Such air in excess is discharged to the outside by means of special safety valves. This notwithstanding, in case of lack of the pressurizing air, due to energy interruption or damage, possible small gas leaks could cause the formation of the explosive mixture. The energy interruption is often due to storms, which at the same time could cause tears to the membranes due to the wind, and this means that such eventuality is not so improbable.

In spherical gas accumulators, the insulation of the air pressurizing chamber from the outside is assured only by the sealing of the lower edge of the second membrane, and possible leaks along said edge cause a bad working of the accumulator.

In particular, the solution of keeping the lower edge of said membranes tightened between two sealing surfaces, obtaining at the same time the hooking to the ground and the impermeable gas sealing, is not satisfactory, because the tie-rods or other fixing means used may easily get loose and compromise the seal.
Furthermore, this is a rather expensive solution, mostly using metal sealing surfaces, that must be machined and cannot be rough; this solution is also rather laborious during the installation, in particular for gas accumulators of relevant dimensions.

Also the solution of realizing the gas accumulation chamber with a close membrane is not completely satisfactory. In fact, at least one gas inlet/outlet flanged connection on the membrane must be provided, and in some cases a plurality of connections must be provided, e.g. when there are two separate gas inlet and gas outlet pipes and a third pipe for the drainage of the steam that condenses in the gas accumulation chamber.
It is evident that the seals of the membrane around said connections are points where gas leaks may occur, that could enter the adjacent air pressurizing.

Also the known devices for measuring the gas quantity contained in the above described accumulation chamber show some inconveniences regarding safety and measuring accuracy.
The use of a cable fixed to the top of the inner membrane has the main inconvenience of mechanically connecting the two membranes by means of said cable. Consequently, stresses of the outer membrane - due to bad weather - are transmitted to the inner membrane and may determine tears or lacerations, with gas leaks to the outside or into the pressurizing chamber. The measuring by means of a ballast on the top of the first membrane and an ultrasonic probe, or any other kind of equivalent probe, prevents the mechanical connection between the two membranes but does not give a completely reliable measure. In fact, the concentrated weight on the first membrane influences the shape of the gas accumulation chamber at a point that, when the ballast touches the resting surface of the gasometer, the latter is not empty. In fact a considerable gas quantity remains in the residual, half-toroidal volume of the gas accumulation chamber around the ballast.

It is the scope of the present invention to eliminate all above described inconveniences and disadvantages.

The main scope of the present invention is to realize a membrane gas accumulator provided with a gas accumulation chamber and a pressurizing chamber adjacent to said gas accumulation chamber, which considerably reduces the risk of gas leaks into the pressurizing chamber, with a great advantage for safety and reliability.
In particular, it is the aim of the present invention to assure that, by means of a passive safety system, possible gas leaks from determined points of the accumulation chamber flow to the outside instead of filtering into the air pressurizing chamber, thus avoiding the risk of fire or explosion of the accumulator itself.

It is a further aim of the present invention to realize a membrane gas accumulator provided with means for hooking the membranes, such as stresses on the same, specially those due to wind and bad weather, may not cause gas infiltrations in the air pressurizing chamber, nor compromise the impermeable sealing of the chambers.

Another aim of the present invention is to realize a membrane gas accumulator provided with a device for measuring the gas content of the accumulation chamber which, in advantage of the safety, does not require a mechanical connection between the two membranes, thus avoiding the risk of tears or lacerations due to the respective movements between said membranes, and that gives an accurate measure of the gas content.

In any case, it is the aim of the present invention to realize a membrane gas accumulator that is cheap, easy in realization and installation, comprising the hooking system to the ground and the device for measuring the gas quantity contained therein.

The aims set forth are reached by means of the present invention, consisting of a flexible membrane gas accumulator, in particular for storing bio-gas, comprising:
- a first membrane at least partially defining a gas accumulation chamber;
- a second membrane partially defining a pressurizing chamber adjacent to said accumulation chamber;
- means for leading and taking gas, connected to said accumulation chamber;
- means for pressurizing said pressurizing chamber by means of auxiliary gas, preferably air;
- means for hooking said first membrane and said second membrane;
- means for detecting the volume of said accumulation chamber;
characterized in that it comprises a third membrane, impermeable to gas and fixed in an impermeable manner at least to said second membrane, for defining, in co-operation with the latter, said pressurizing chamber.

The main advantage obtained by means of the present invention consists in that, due to the presence of the third membrane that defines the pressurizing chamber in co-operation with the second membrane, the insulation degree between the two chambers is increased and the risk is reduced that gas leaks from the accumulation chamber filter into the pressurizing chamber.
Said third membrane, in fact, forms a further barrier to the passage of gas from one chamber to the other. Possible gas leaks from the accumulation chamber remain restricted in the space between the first and the third membrane, from where they can flow to the outside, because the pressure in said space, at direct contact with the atmosphere, is considerable lower that the pressure of the gas and than the pressure of the air in the pressurizing chamber.

A further advantage consists in that, due to the presence of said third membrane, it is not necessary that the ground hookings of the first and the third membrane have a seal impermeable to gas. Said hookings may be exclusively of a mechanical kind and therefore cheaper and easier to realize, with the further advantage that the stresses on said hookings do not compromise the seal.
In particular, for spherical and cylindrical gasometers, if the ground hooking of the second membrane is not impermeable to gas, the discharge to the atmosphere of possible gas leaks from the accumulation chamber is allowed.
The hooking of the first membrane may also be permeable to the gas, if said membrane is closed or its edges are immersed in a liquid.

For obtaining such a hooking - at the same time cheap and easy to realize - it may be advantageous to provide the membranes with hooking edges, connected with fixed supports disposed at a suitable distance one from another.

For realizing an easy and accurate system for measuring the gas content of the accumulation chamber without any mechanical connection between the membranes, the detecting means comprise means for measuring the weight of an element suspended above said first membrane, that said first membrane, rising or lowering under the pressure of the gas, pushes towards the top or releases.

These and other advantages will be more evident from the enclosed specification of the present invention, that will be described hereinbelow relating to the enclosed drawings in which some preferred embodiments are shown.

Figure 1 shows a cross section of a membrane gas accumulator, of the kind with a spherical or cylindrical dome, according to the present invention.

Figure 2 shows a cross section of a detail of the ground hooking of the gas accumulator according to figure 1.

Figure 3 shows a variant of the ground hooking shown in figure 2.

Figure 4 shows a front view of the hooking of figure 2.

Figure 5a shows a cross section of a detail of a hooking similar to the one of figure 2, only for the gas chamber, in a variant that may be applied if the first membrane is open on the bottom.

Figure 5b is similar to figure 5a and relates to a dome accumulator in which the gas accumulation chamber is closed at the bottom by a liquid surface.

Figure 6 shows a cross section of a variant of the accumulator according to figure 1.

Figure 7 shows a cross section of a closed-cylinder membrane gas accumulator, realized according to the present invention.

Figure 8 shows in detail a cross section and a perspective view of the fixing and hooking of the membranes of the gas accumulator of figure 7.

Figures 9a and 9b show a schematic cross section of a membrane accumulator according to the present invention, respectively empty and partially filled with gas.

Relating now to the details shown in the figures, the gas accumulator according to the present invention comprises a first membrane 1 and a second membrane 2. Said membrane 1 defines a gas accumulation chamber C1, while said membrane 2 partially defines a pressurizing chamber C2.

The chamber C1 is closed at the bottom by a base surface S, impermeable to gas. Said surface S may advantageously consist of a concrete impermeable basement, of said membrane 1 itself, or by the free surface of the liquid contained in a basin, for the covering of which said membrane 1 is provided.

Means 3 for leading and taking gas are connected to said chamber C1, while pressurizing means 4 are connected to said chamber C2. If the surface S is a liquid surface from which the gas exhales that is collected directly in said chamber C1, said means 3 serve only for taking the gas.

Said means 3 comprise special pipes and flanged connections on the edge of said membrane 1, while said pressurizing elements 4 advantageously comprise a fan or air compressor and pipes connected to the surface of said membrane 2. Further accessory elements belonging to the known art, like exhaust-valves and safety valves for air and gas, are not shown.

Both membranes 1 and 2 are hooked to a basement B by means of mechanical hooking means 5.

Detecting means 6, adapted to detect the volume of the gas accumulation chamber C1, are provided on the top of the accumulator, to allow measuring of the gas content of said chamber C1.

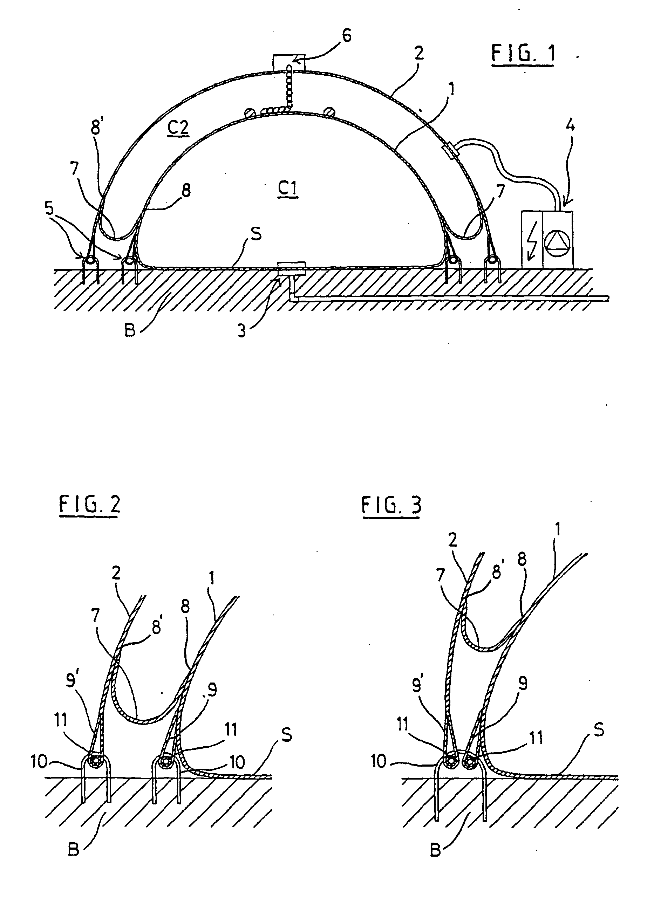

As shown in figure 1, said accumulator comprises a third membrane 7 fixed in an impermeable manner to said membrane 1 along an edge 8, and fixed to said membrane 2 along an edge 8'. The pressurizing chamber C2 is therefore defined by said membrane 1, by said membrane 2 and by said membrane 7.

The hooking means 5 comprise hooking edges 9 and 9', respectively connected to said membranes 1 and 2, connected to rings 10 fixed to the basement B by means of rigid tubular elements 11 which enter edges 9 and 9' as well as rings 10. The latter are arranged on two ranks, an inner rank for the hooking of said membrane 1 and an outer rank for the hooking of said membrane 2. Figure 3 shows a variant in which sad rings 10 are arranged on a single rank.

The rigid tubular elements 11 and the rings 10 are advantageously made of steel, while the hooking edges 9 and 9' are made of a material similar to the one of said membranes 1 and 2, or obtained in said membranes themselves.

This hooking system is not impermeable to the gas, because the edges 9 and 9' are not continuous but have slits 12 in correspondence of the rings 10, and openings 13 below the tubular elements 11.

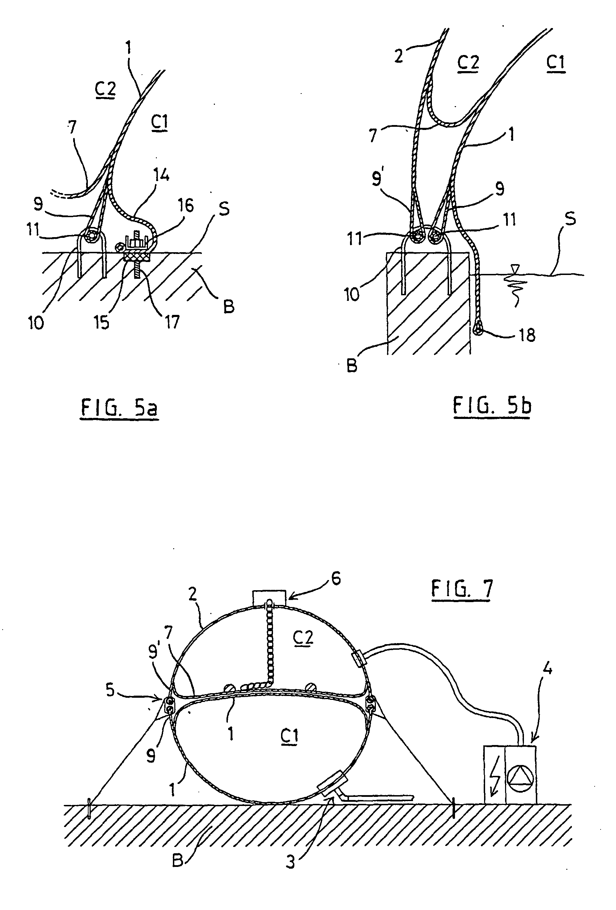

Figure 5a shows a possible variant of the described hooking system, that may be used for a dome gas accumulator, wherein the membrane 1 must be impermeably sealed to base surface S.
In the shown example, this is obtained by tightening one portion 14 of said membrane 1 to special sealing means 15, which act as gas impermeable seals by means of a section 16 and special tie-rods 17. As the traction force on the membrane 1 is absorbed by the hooking of the edges 9 and is not transmitted to the portion 14, the seal obtained in this way is not negatively influenced by the stresses on the membrane 1, that is an advantage for safety.

It is evident that, in combination with the above described mechanical hooking system by means of said edge 9, any other impermeable system for fixing the edge of said membrane 1 to said surface S may be used, obtaining the same advantages.

In figure 5b, the base surface S of the gas accumulation chamber is given by the free surface of a liquid, and the seal along the lower edge of said membrane 1 is obtained by keeping said edge immersed in said liquid, with the help of a ballast element 18, consisting of a steel reinforcing rod.

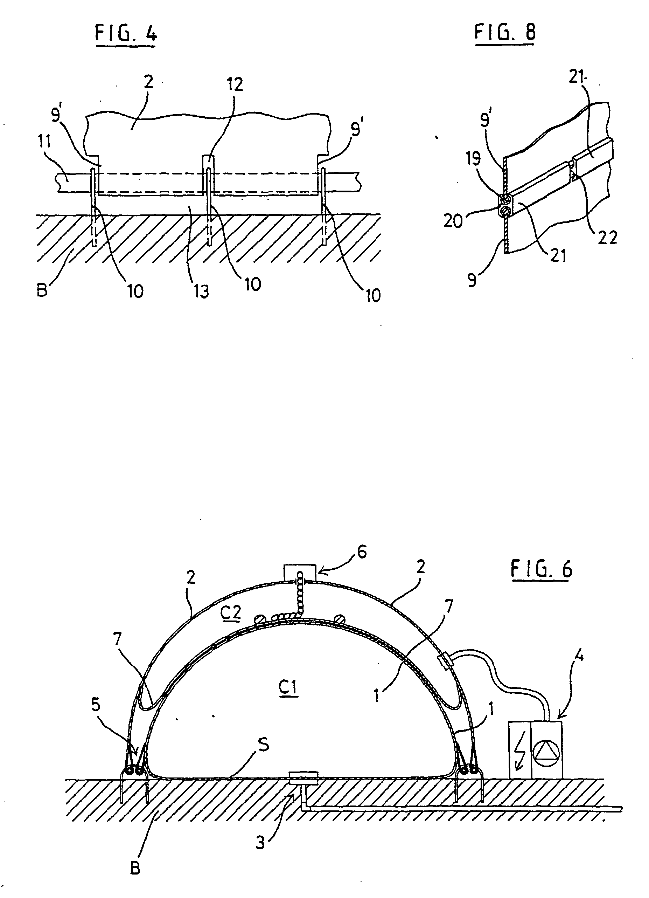

Figure 6 shows a variant of the present invention, in which said membrane 7 is fixed only to said membrane 2 along the edge 8'. In this case, the pressurizing chamber C2 is defined by the membrane 2 and by the membrane 7, and said membrane 7 is in contact with part of the surface of said membrane 1, so that the chambers C1 and C2 result in being adjacent.
The membrane 7 is advantageously realized in such a manner as to rest onto membrane 1 in a way permeable to gas, to allow possible gas leaks from any point of the gas accumulation chamber C1 to flow between the two membranes.
For this purpose, the membrane 1 may be realized with PVC-coated fabric, on both sides, while the membrane 7 is also made of fabric, but the side in contact with the membrane 1 is coated with a material different from PVC having some lubricating characteristics, advantageously silicon, so as to prevent the membranes from adhere one to the other.
As an alternative, the surface of the membrane 7 in contact with the membrane 1 may be provided with special projections or grooves for obtaining a support permeable to gas on said membrane 1.

The hooking means 5 - in this variant of the present invention as well - are realized in a manner similar to what above described, with the possibility of the same variants.

Figure 7 shows the application to the present invention to closed-cylinder accumulators.

The membrane 1 forms a closed accumulation chamber C1, having a mainly cylindrical shape, while the membrane 2, fixed to said membrane 1 along two external, approximately diametrically opposed edges, delimits a pressurizing chamber C2 adjacent to chamber C1, for its whole length.

Figure 8 shows a variant of the hooking means 5, that may be applied in particular to this kind of accumulators.
In this variant, the edges 9 and 9' of the membranes 1 and 2 are tied around retention ropes 19 passing through blocking seats 20 obtained by means of sections 21. In this case as well, openings 22, similar to said openings 12 and 13, are provided between the edges of the membranes and said sections 21. Said sections 21 are advantageously H-shaped or double C-shaped so as to fit in the retaining ropes 19 of both membranes.

In a further variant, not shown, the retention ropes 19 may be passing through rings applied to the edges of the membranes, or through eyelets realized in the edges of said membranes.

The detecting means 6, at last, comprise a chain 23, having one end hanging from a device 24 adapted to measure the weight of said chain, and the other end being freely resting on the top of the chamber C1.
Said device 24 advantageously comprises a loading cell.

An edge 25 is provided on the top of the chamber C1, delimiting a resting area 26 for the free end of the chain 23.

During the working of the gas accumulator, the chamber C1 is filled or emptied, thus varying its shape and volume, while the chamber C2 is kept at a determined pressure level by said pressurizing means 4.

The pressure of the chamber C2 onto the adjacent chamber C1 allows the delivery of the gas at the desired pressure and facilitates the emptying of the accumulation chamber C1.

Possible gas leaks from the chamber C1 to the space comprised between the membrane 1, the membrane 2 and the membrane 7 are released directly into the atmosphere through the openings 12, 13 or 22, thus preventing the filtering of the gas into the chamber C2 and the risk of fire or explosion.

In particular, in the solution shown in figure 1, possible gas leaks are delivered to the outside from the lower portion of the membrane 1, i.e. the portion below the edge 8, where there is a greater risk that gas leaks may occur, in particular due to the presence of the flanges of the gas inlet and outlet pipes and for the water drainage, usually located just in the bottom part of the chamber C1.

In the variant according to figure 6 and in the application according to figure 7, on the other hand, as no point of the membrane 1 directly separates the chambers C1 and C2, due to the interposition of the membrane 7, any gas leak from the chamber C1 is released into the atmosphere and has no way to enter the chamber C2.

As the membrane 7 gets placed onto said membrane 1 in a gas permeable manner, a gas leak from that point of said membrane 1 onto which said membrane 7 is resting may flow down, as well, passing between the two membranes towards one of the outside leading openings.

Said edges 9 and 9' hooked to said rings 10 balance the traction forces on the membranes, including the forces due to the wind, but at the same time they leave the membranes sufficiently free to move and to get deformed and they do not create a constraint that may tear or lacerate the membranes.

Furthermore, the hooking solution as described above prevents the stresses on the hookings from compromising the seal between said chambers C1 and C2 and towards the outside, as the hookings of said edges 9 and 9' are only mechanical and do not have sealing purposes.

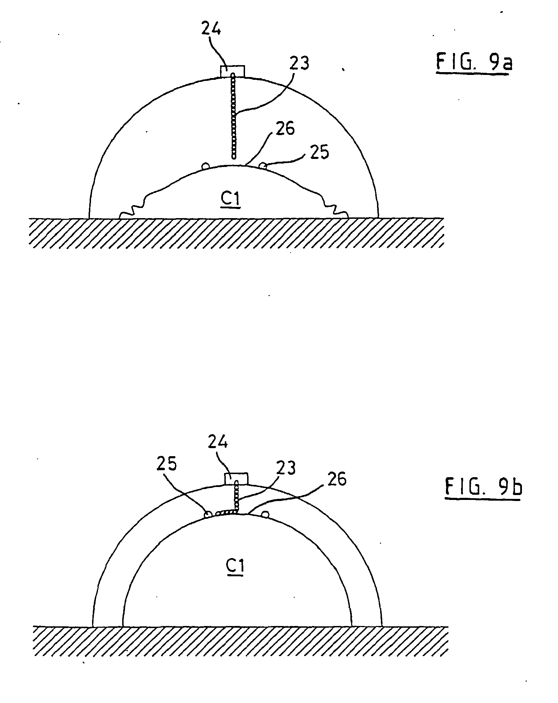

The working of the detecting means 6, as shown in figures 9a and 9b, is the following.

When the free end of said chain 23 does not touch the top of the chamber C 1, the device 24 measures the total weight of said chain.
On the contrary, when the chamber C1 is partially or completely filled up with gas, a portion of the chain 23 rests on the top of said chamber and the device 24 measures a tension only due to the weight of that portion of the chain that remains suspended.
Thus, the measure given by the device 24 is an indirect measure of the quote assumed by the top of the chamber C1. This measure may be easily geometrically correlated to the volume of said chamber C1 and therefore to the volume of stored gas, as the shape of the chamber C1 is not influenced by the weight of the chain 23 and substantially remains spherical in any filling condition, in particular when said chamber is almost empty and the chain 23 is substantially completely hanging from said device 24.

The edge 25 keeps the chain 23 resting in an area 26, avoiding the lateral sliding of said chain, that could compromise the accuracy of the measuring.

Said chain 23 can not even transmit traction stress to the membrane 1, that could be dangerous for the integrity of said membrane, because it rests onto he top of the chamber C1 without being fixed.

In the boundary of the present invention, the chain 23 may be obviously replaced by an element performing the same function, e.g. a rope heavy enough or a rope made heavy by loads placed along the same at regular distances.

In a variant not shown, the device 24 may be supported by an external fixing element, instead by the top of said membrane 2.

## Claims

1. A flexible membrane gas accumulator, in particular for storing bio-gas, comprising:
- a first membrane (1) at least partially defining a gas accumulation chamber (C1);
- a second membrane (2) partially defining a pressurizing chamber (C2) adjacent to said accumulation chamber (C1);
- means (3) for leading and taking gas, connected to said accumulation chamber (C1);
- means (4) for pressurizing said pressurizing chamber (C2) by means of an auxiliary gas, preferably air;
- means (5) for hooking said first membrane (1) and said second membrane (2);
- means (6) for detecting the volume of said accumulation chamber (C1);
***characterized in that*** it comprises a third membrane (7), impermeable to gas and fixed in an impermeable manner at least to said second membrane (2), for defining, in co-operation with the latter, said pressurizing chamber (C2).

2. A gas accumulator according to claim 1, ***characterized in that*** said third membrane (7) is fixed in an impermeable manner to said first membrane (1) and to said second membrane (2), respectively along a first edge (8) and a second edge (8') of said third membrane (7), so that said pressurizing chamber (C2) is defined by a portion of said first membrane (1) and by said third membrane (7).

3. A gas accumulator according to claim 1, ***characterized in that*** said third membrane (7) is fixed in an impermeable manner to said second membrane (2) along an edge (8'), so that said pressurizing chamber (C2) is defined only by said second membrane (2) and by said third membrane (7).

4. A gas accumulator according to claim 1, ***characterized in that*** said third membrane (7) is resting in contact on at least one portion of the surface of said first membrane (1), in a gas permeable manner.

5. A gas accumulator according to at least one of the preceding claims, ***characterized in that*** at least one of said membranes (1, 2) comprise hooking edges (9, 9') for the connection to fixing means.

6. A gas accumulator according to claim 5, ***characterized in that*** said fixing means comprise linear rigid elements (11) passing through said hooking edges (9, 9') and through fixed rings (10), arranged in one or more ranks.

7. A gas accumulator according to claim 5, ***characterized in that*** said hooking edges (9, 9') comprise at one end a fixing rope (19) that may be inserted in a blocking seat (20) of a section (21).

8. A gas accumulator according to claim 1, ***characterized in that*** said means (6) for detecting the volume of the accumulation chamber (C 1) comprise a chain (23) having one free end and the other end hanging from a device (24) adapted to measure the weight of said chain (23), and said device (24) is supported at the top of said second membrane (2).

9. A gas accumulator according to claim 8, ***characterized in that*** the top of the gas accumulation chamber (C1) comprises an edge (25) that defines an area (26) for the support of the free end of said chain (23).

10. A gas accumulator according to one or more of the preceding claims, ***characterized in that*** said first and second membrane (1, 2) have substantially the shape of a spherical or cylindrical dome.

## Patentansprüche

1. Flexibler Membrane-Gasspeicher, insbesondere für die Speicherung von Biogas, bestehend aus:
- einer ersten Membrane (1), die mindestens teilweise einen Gasspeicherraum (C1) bestimmt;
- einer zweiten Membrane (2), die teilweise einen neben dem genannten Gasspeicherraum (C1) liegenden Druckraum (C2) bestimmt;
- Mitteln (3) zum Leiten und Ansaugen von Gas, verbunden mit genanntem Speicherraum (C1);
- Mitteln (4), um genannten Druckraum (C2) mit Hilfe von Zusatzgasen, vorzugsweise Luft, unter Druck zu setzen;
- Mitteln (5) zum Anhaken der genannten ersten (1) und der genannten zweiten (2) Membrane;
- Mitteln (6) zum Messen des Volumens des genannten Speicherraumes (C1);
**dadurch gekennzeichnet, dass** er eine dritte gasundurchlässige Membrane (7) umfasst, die auf undurchlässige Weise mindestens an genannte zweite Membrane (2) befestigt ist, sodass unter Mitwirkung letzterer der genannte Druckraum (C2) bestimmt wird.

2. Gasspeicher nach Anspruch 1, **dadurch gekennzeichnet, dass** genannte dritte Membrane (7) auf undurchlässige Weise an genannte erste Membrane (1) und an genannte zweite Membrane (2) entlang einer ersten Kante (8) bzw. einer zweiten Kante (8') der genannten dritten Membrane (7) befestigt ist, so dass der genannte Druckraum (C2) durch einen Teilbereich der genannten ersten Membrane (1) und durch genannte dritte Membrane (7) bestimmt wird.

3. Gasspeicher nach Anspruch 1, **dadurch gekennzeichnet, dass** genannte dritte Membrane (7) auf undurchlässige Weise an genannte zweite Membrane (2) entlang einer Kante (8') befestigt ist, so dass genannter Druckraum (C2) nur durch die genannten zweite Membrane (2) und durch genannte dritte Membrane (7) bestimmt wird.

4. Gasspeicher nach Anspruch 1, **dadurch gekennzeichnet, dass** genannte dritte Membrane (7) in Berührung auf mindestens einem Teilbereich der Oberfläche der genannten ersten Membrane (1) auf gasdurchlässige Weise aufliegt.

5. Gasspeicher nach mindestens einem der vorstehenden Ansprüche, **dadurch gekennzeichnet, dass** mindestens eine der genannten Membranen (1, 2) Einhängekanten (9, 9') für die Verbindung an die Befestigungsmitteln umfasst.

6. Gasspeicher nach Anspruch 5, **dadurch gekennzeichnet, dass** genannte Befestigungsmittel lineare starre Elemente (11) umfassen, die durch genannte Einhängekanten (9, 9') und durch ortsfeste Ringe (10) laufen, angebracht in einer oder mehreren Reihen.

7. Gasspeicher nach Anspruch 5, **dadurch gekennzeichnet, dass** genannte Einhängekanten (9, 9') an einem Ende ein Befestigungsseil (19) umfassen, das in einen Sperrsitz (20) eines Profilquerschnittes (21) eingesetzt werden kann.

8. Gasspeicher nach Anspruch 1, **dadurch gekennzeichnet, dass** genannte Mitteln (6) zum Messen des Volumens des Speicherraumes (C1) eine Kette (23) umfassen mit einem freien Ende und einem anderen Ende, das aus einer zum Messen des Gewichtes der genannten Kette (23) geeigneten Vorrichtung (24) heraushängt und dass genannte Vorrichtung (24) am oberen Ende der genannten zweiten Membrane (2) gelagert ist.

9. Gasspeicher nach Anspruch 8, **dadurch gekennzeichnet, dass** das obere Ende des Gasspeicherraumes (C1) eine Kante (25) umfasst, die einen Bereich (26) für die Auflage des freien Ende der genannten Kette (23) bestimmt.

10. Gasspeicher nach einem oder mehreren vorstehenden Ansprüchen, **dadurch gekennzeichnet, dass** genannte erste und zweite Membrane (1, 2) im wesentlichen die Form einer kugelförmigen oder zylindrischen Kuppel haben.

## Revendications

1. Un accumulateur de gaz à membrane flexible, notamment pour le stockage de biogaz, comprenant :
- une première membrane (1) définissant au moins partiellement une chambre d'accumulation de gaz (C1) ;
- une deuxième membrane (2) définissant partiellement une chambre de pressurisation (C2) adjacente à ladite chambre d'accumulation (C1);
- un dispositif (3) pour conduire et prélever le gaz, connecté à ladite chambre d'accumulation (C1) ;
- un dispositif (4) pour pressuriser ladite chambre de pressurisation (C2) à l'aide d'un gaz auxiliaire, de préférence de l'air ;
- un dispositif (5) pour accrocher ladite première membrane (1) et ladite deuxième membrane (2) ;
- un dispositif (6) pour détecter le volume de ladite chambre d'accumulation (C1) ;
**caractérisé par le fait qu'**il comprend une troisième membrane (7), imperméable au gaz et fixée de manière imperméable au moins à ladite deuxième membrane (2), pour définir, en coopération avec cette dernière, ladite chambre de pressurisation (C2).

2. Un accumulateur de gaz selon la revendication 1, **caractérisé par le fait que** ladite troisième membrane (7) est fixée de manière imperméable à ladite première membrane (1) et à ladite deuxième membrane (2), respectivement le long d'un premier bord (8) et d'un deuxième bord (8') de ladite troisième membrane (7), de sorte que ladite chambre de pressurisation (C2) est définie par une portion de ladite première membrane (1) et par ladite troisième membrane (7).

3. Un accumulateur de gaz selon la revendication 1, **caractérisé par le fait que** ladite troisième membrane (7) est fixée de manière imperméable à ladite deuxième membrane (2) le long d'un bord (8'), de sorte que ladite chambre de pressurisation (C2) est définie uniquement par ladite deuxième membrane (2) et par ladite troisième membrane (7).

4. Un accumulateur de gaz selon la revendication 1, **caractérisé par le fait que** ladite troisième membrane (7) repose en contact sur au moins une portion de la surface de ladite première membrane (1), d'une manière perméable au gaz.

5. Un accumulateur de gaz selon au moins l'une des revendications précédentes, **caractérisé par le fait qu'**au moins une desdites membranes (1, 2) comprend des bords d'accrochage (9, 9') pour la connexion au dispositif de fixation.

6. Un accumulateur de gaz selon la revendication 5, **caractérisé par le fait que** ledit dispositif de fixation comprend des éléments rigides linéaires (11) passant à travers lesdits bords d'accrochage (9, 9') et à travers des bagues fixes (10), disposées sur un ou plusieurs rangs.

7. Un accumulateur de gaz selon la revendication 5, **caractérisé par le fait que** lesdits bords d'accrochage (9, 9') comprennent à une extrémité une corde de fixation (19) qui peut être insérée dans un siège de blocage (20) d'une section (21).

8. Un accumulateur de gaz selon la revendication 1, **caractérisé par le fait que** ledit dispositif (6) pour détecter le volume de la chambre d'accumulation (C1) comprend une chaîne (23) ayant une extrémité libre et l'autre extrémité pendant depuis un dispositif (24) prévu pour mesurer le poids de ladite chaîne (23), et ledit dispositif (24) est supporté au-dessus de ladite deuxième membrane (2).

9. Un accumulateur de gaz selon la revendication 8, **caractérisé par le fait que** le dessus de la chambre d'accumulation de gaz (C1) comprend un bord (25) qui définit une zone (26) pour le support de l'extrémité libre de ladite chaîne (23).

10. Un accumulateur de gaz selon une ou plusieurs des revendications précédentes, **caractérisé par le fait que** lesdites première et deuxième membranes (1, 2) ont substantiellement la forme d'un dôme sphérique ou cylindrique.
